# EUROPEAN PATENT SPECIFICATION

(11) **EP 2 471 795 B1**
(45) Date of publication and mention of the grant of the patent: **20.04.2016**
(21) Application number: 10811187.3
(22) Date of filing: 23.06.2010
(51) Int. Cl.: C07D 495/04

(54) **METHOD FOR PREPARING PRASUGREL**
VERFAHREN ZUR HERSTELLUNG VON PRASUGREL
PROCÉDÉ POUR LA PRÉPARATION DE PRASUGREL

(30) Priority: 26.08.2009 CN 200910170675
(43) Date of publication of application: 04.07.2012
(73) Proprietor: Zhejiang Huahai Pharmaceutical Co., Ltd., Zhejiang 317-024 (CN)
(72) Inventor: LIU, Chuangwei, Linhai Zhejiang 317024 (CN); LU, Qifeng, Linhai Zhejiang 317024 (CN); CHEN, Changhui, Linhai Zhejiang 317024 (CN)
(74) Representative: Kling, Simone
(86) International application number: PCT/CN2010/074310
(87) International publication number: WO 2011/023027

(56) References cited:
- EP-A1- 2 003 136
- EP-A1- 2 123 656
- EP-A2- 0 542 411
- WO-A1-2008/108291
- WO-A2-2009/062044
- WO-A2-2009/066326
- CN-A- 1 074 446
- CN-A- 101 472 929
- BRITTAIN: "X-RAY DIFFRACTION III: PHARMACEUTICAL APPLICATIONS", SPECTROSCOPY, ELSEVIER, AMSTERDAM, NL, vol. 16, no. 7, 1 July 2001 (2001-07-01), pages 14-18, XP002606497, ISSN: 0712-4813

## Description

### FIELD OF THE INVENTION

The present invention belongs to the pharmaceutical and chemical field, in particular, relates to a preparation method of prasugrel and crystallization method thereof.

### BACKGROUND OF THE INVENTION

Prasugrel is an oral anti-platelet drug co-developed by Eli Lilly and its partner Daiichi Sankyo Co. Ltd. It has been reported that prasugrel hydrochloride and prasugrel maleate both have good anti-thrombotic activies and are a new generation of potent thienopyridines anti-platelet drug, and thus much attention has been paid to the synthesis of prasugrel compounds. The structural formula of prasugrel is as follows:

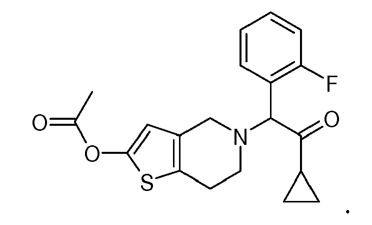

Several methods for synthesizing prasugrel in the form of free base were reported in European Patent No. 0542411, US Patent No.20030134872 and European Patent No.0785205. All of these methods comprise firstly condensating α-cyclopropylcarbonyl-2-fluorobenzyl halide (compound 2) with 2-oxo-4,5,6,7-tetrahydrothieno[3,2-c]pyridine salt (compound 3) to obtain 5-(α-cyclopropylcarbonyl-2-fluorobenzyl)-2-oxo-2,4,5,6,7-tetrahydrothieno[3,2-c]pyridine (compound 4), and compound 4 is finally acylated and purified to give prasugrel (compound 5 ). WO2009/066326 discloses a process for preparing crystalline prasugrel wherein the precursors are reacted with NBS in chloroform and the raw prasugrel is purified from ethyl acetate/cyclohexane.

However, the processes provided in the above patents have the following defects: (1) the yield for the synthetic step of intermediate compound 4 is low (only 32% to 35%); (2) the cost of the brominating agent NBS adopted is high, moreover, the toxicity of the solvents used such as carbon tetrachloride or chloroform is relatively high, and are not good for the environmental protection; and (3) the target compound is obtained by final purification through column chromatography, as a result, the separation method is complicated and the cost of separation is high. Thus, these processes are not suitable for large-scale industrial production.

### SUMMARY OF THE INVENTION

The purpose of the present invention is to provide a synthesis method which is safe, economic and easy to operate and purify, and has a high yield, thus is suitable for large-scale industrial production.

To achieve this purpose, the technical solutions used in the present invention are as follows:
The X-ray polycrystalline diffraction pattern of prasugrel crystal powder has the following characteristic diffraction peaks (2θ): 7.63±0.2, 11.11±0.2, 13.34±0.2, 14.63±0.2, 18.48±0.2, 18.76±0.2, 19.22±0.2, 20.64±0.2, 21.30±0.2, 22.64±0.2, 23.30±0.2, 24.68±0.2, 26.22±0.2, 26.86±0.2, 29.42±0.2 and 31.28±0.2.

The melting point of the crystalline form as mentioned above is 121.3°C∼125.2°C.

A method for synthesizing crystalline form A of prasugrel with the above diffraction pattern comprised the following steps:
(1) o-fluorobenzyl cyclopropyl ketone as a raw material is reacted with 1,3-dibromo-5,5-dimethylhydantoin (DBDMH) or 1,3-dichloro-5,5-dimethylhydantoin as a halogenating agent in acetic acid as a solvent at a temperature of 0 °C ∼100 °C to give α-cyclopropylcarbonyl-2-fluorobenzyl halide (compound 2).
   Preferably, at a temperature of 60°C∼85°C, α-cyclopropylcarbonyl-2-fluorobenzyl halide (compound 2) is mildly formed using o-fluorobenzyl cyclopropyl ketone as raw material, 1,3-dibromo-5,5-dimethylhydantoin or 1,3-dichloro-5,5-dimethylhydantoin as a halogenating agent, and acetic acid as a solvent by addition in dropwise, thus the outbreak of free radical reaction is avoided. The molar ratio of O-fluorobenzyl cyclopropyl ketone to the halogenating agent is 1:0.5-0.65, preferably 1:0.6. Compared with the process used in US.Pat.No. 20030134872, the operating conditions are greatly improved by using 1,3-dibromo-5,5-dimethylhydantoin as a halogenating agent, and the selectivity and efficiency of halogenation are greatly improved. Environmental pollution is reduced and the degree of environmental protection is increased by using acetic acid as a solvent compared to the process using chloroform.
(2) α-cyclopropylcarbonyl-2-fluorobenzyl halide (compound 2) is then reacted with 2-oxo-4,5,6,7-tetrahydrothieno[3,2-c]pyridine p-toluenesulfonate (compound 3) in the concerted catalysis of a phase transfer catalyst and an inorganic salt using an inorganic base as an acid binding agent and DMF as a solvent under nitrogen atmosphere to produce 5-(α-cyclopropylcarbonyl-2-fluorobenzyl)-2-oxo-2,4,5,6,7-tetrahydrothieno[3,2-c]pyridine (compound 4).
   In this step, the molar ratio of compound 2 to compound 3 is 1:1-1.5, preferably 1:1.15. The molar ratio of compound 2 to the inorganic alkali is 1:2-8, preferably 1:4. The inorganic alkali can be one or more selected from the group consisting of sodium carbonate, potassium carbonate, sodium bicarbonate and potassium bicarbonate, more preferably sodium bicarbonate. The molar ratio of compound 2 to the phase transfer catalyst is 1:0.05-1.2, preferably 1:1.01. The phase transfer catalyst can be selected from quaternary ammonium salts, such as tetrabutyl ammonium bromide, tetraethyl ammonium bromide, tetrabutyl ammonium chloride, tetraethyl ammonium chloride and the like. The molar ratio of compound 2 to the inorganic salt is 1:0.02-1.2, preferably 1:0.1. The inorganic salt includes bromides, such as sodium bromide and potassium bromide.
   In the above step, the temperature of the catalytic reaction is usually 0°C∼100°C, preferably 40°C∼50°C.
   A catalytic amount of a phase transfer catalyst is used in combination with an inorganic salt to perform a concerted catalysis in this step. Inorganic salts such as sodium bromide may have a catalytic or salt effect on the nucleophilic substitution reaction in this reaction process. Compared with the process described in US.Pat.No. 20030134872, the reaction yield is improved to 90% and the reaction time is shortened to 45 minutes.
(3) Compound 4 is acylated in the presence of an acylating agent using triethylamine as an acid binding agent and DMF as a solvent under nitrogen atmosphere at room temperature, and finally extracted, dried and filtrated to give the mother liquor of prasugrel. The molar ratio of compound 4 to triethylamine in this step is 1:1-10, preferably 1:3. The acylating agent used can be selected from acetic anhydride or acetyl chloride, preferably acetic anhydride.
(4) An alcoholic solvent is added to the prasugrel gum obtained in the above step, a crystal is precipitated after stirring at room temperature. The crystal is filtrated to give prasugrel solid. The alcoholic solvents include one or more selected from the group consisting of aliphatic alcohols such as methanol, ethanol, isopropanol, n-butanol, tert-butanol, benzyl alcohol and aromatic alcohols such as benzyl alcohol. The alcoholic solvent is preferably methanol, ethanol and isopropyl alcohol.

The ratio of the volume of the alcoholic solvent used to the weight of α-cyclopropylcarbonyl-2-fluorobenzyl halide is 2.5ml/g-6.5ml/g, preferably 3.0ml/g.

The purity of prasugrel obtained in this step is ≥95%. The product is recrystallized once again from ethanol to get a white crystal whose purity is ≥99.50%.

In this step, an alcoholic solvent as the crystallization and recrystallization solvent is used for the final separation and purification of prasugrel, to give the title compound by crystallize and purification. The separation method of the present invention is more simple, feasible, economic and favorable for large-scale industrial production compared with the separation and purification process (column chromatography) in US Patent No. 20030134872.

The reaction scheme of the invention is as follows:

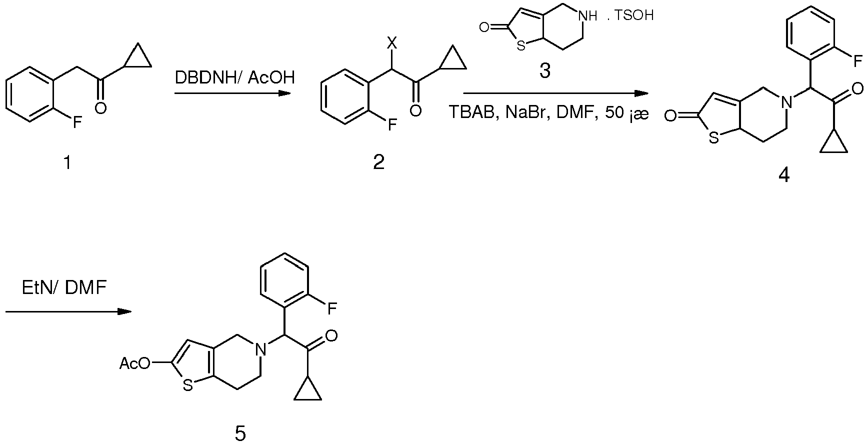

The synthesis method for prasugrel provided in the present invention employs an alcoholic solvent as the crystallization and recrystallization solvent for crystallization and purification and the yield of the reaction is high. The method has the advantages of safety, economy, low-pollution and simple operation, thus, it is suitable for large-scale industrial production.

### BRIEF DESCRIPTION OF THE DRAWINGS

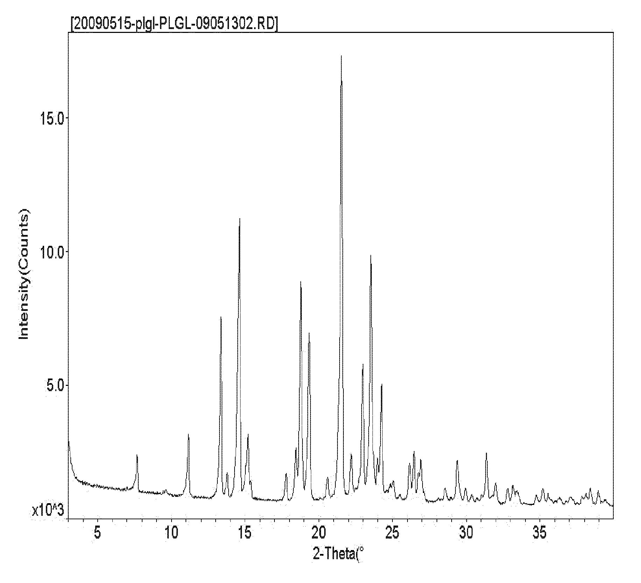

Figure 1 is the X-ray powder diffraction pattern of crystalline form A of prasugrel powder crystal. The lateral axis represents the diffraction angle (2θ), and the vertical axis represents intensity (CPS).

### DETAILED DESCRIPTION OF THE INVENTION

Now the present invention will be further illustrated in combination with the following examples, so that those skilled in the art can understand the present invention better. However, the scope of the present invention is not limited in any way.

### Example 1:

### Preparation of α-cyclopropylcarbonyl-2-fluorobenzyl bromide (compound 2) :

13.1g o-fluorobenzyl cyclopropyl ketone and 40 ml acetic acid were added to a 100ml four-neck flask equipped with a mechanical stirring device, a thermometer, a reflux condenser and a constant-pressure dropping funnel. 12.1g 1,3-dibromo-5,5-dimethylhydantoin and 0.66g azobisisobutyronitrile were added in dropwise to the above reaction system at 60°C∼85°C over 2 hours. After the completion of addition, the mixture was kept at this temperature and stirred for 25 minutes. Next, the mixture was cooled and distilled to remove most of acetic acid. 40ml ethyl acetate and 40ml water were added to the concentrated solution, and the system was allowed to seperation. The organic layer was washed with 20ml saturated Na₂SO₃, 20ml saturated NaHCO₃ and 20ml saturated brine successively, and then dried over anhydrous magnesium sulfate. The filtrate was distillated under reduced pressure and concentrated to give 21.1g brown oil. The yield was 83.5% and the purity was 74.8%.

### Example 2:

### Preparation of α-cyclopropylcarbonyl-2-fluorobenzyl chloride (compound 2) :

13.1g o-fluorobenzyl cyclopropyl ketone and 40 ml acetic acid were added to a 100ml four-neck flask equipped with a mechanical stirring device, a thermometer, a reflux condenser and a constant-pressure dropping funnel. 9.9g 1,3-dibromo-5,5-dimethylhydantoin was added in dropwise to the above reaction system over 0.5 hour. After the completion of addition, the mixture was stirred for 2 hours at room temperature. Next, the mixture was cooled and distilled to remove most of acetic acid. 40ml ethyl acetate and 40ml water were added to the concentrated solution, and the system was allowed to seperation. The organic layer was washed with 20ml saturated Na₂SO₃, 20ml saturated NaHCO₃ and 20ml saturated brine successively, and then dried over anhydrous magnesium sulfate. The filtrate was distillated under reduced pressure and concentrated to give 16.6g brown oil. The yield was 79.5% and the purity was 75.0%.

### Example 3:

### Preparation of 5-(α-cyclopropylcarbonyl-2-fluorobenzyl)-2-oxo -4,5,6,7-tetrahydrothieno[3,2-c]pyridine (compound 4)

25.7g α-cyclopropylcarbonyl-2-fluorobenzyl bromide prepared in Example 1, 37.6g 2-oxo-4,5,6,7-tetrahydrothieno[3,2-c]pyridine p-toluenesulfonate, 33.6g sodium bicarbonate, 1.0g sodium bromide, 3.2g tetrabutylammonium bromide and 150ml DMF were added to a 1000ml four-neck flask equipped with a mechanical stirring device, a thermometer, a reflux condenser and a constant-pressure dropping funnel. The mixture was stirred for 45 minutes under nitrogen atmosphere at 40°C∼55°C and cooled. 200ml ethyl acetate and 300ml water were added thereto, and the system was allowed to seperation. The organic layer was washed with 300ml water and 100ml saturated brine successively, and then dried over anhydrous magnesium sulfate. The resulting mixture was decolored with silica gel bed and filtered. The filtrate was distillated under reduced pressure and concentrated to give 26.75g brown gum. The yield was 94.5% and the purity was 87.46%.

### Example 4:

### Preparation of 5-(α-cyclopropylcarbonyl-2-fluorobenzyl)-2-oxo -4,5,6,7-tetrahydrothieno[3,2-c]pyridine (compound 4)

25.7g α-cyclopropylcarbonyl-2-fluorobenzyl bromide prepared in Example 1, 37.6g 2-oxo-4,5,6,7-tetrahydrothieno[3,2-c]pyridine p-toluenesulfonate, 40.0g potassium bicarbonate, 8.24g sodium bromide, 3.2g tetrabutylammonium bromide and 150ml DMF were added to a 1000ml four-neck flask equipped with a mechanical stirring device, a thermometer, a reflux condenser and a constant-pressure dropping funnel. The mixture was stirred for 45 minutes under nitrogen atmosphere at 40°C∼55°C and cooled. 200ml ethyl acetate and 300ml water were added thereto, and the system was allowed to seperation. The organic layer was washed with 300ml water and 100ml saturated brine successively, and then dried over anhydrous magnesium sulfate. The resulting mixture was decolored with silica gel bed and filtered. The filtrate was distillated under reduced pressure and concentrated to give 27.10g brown gum. The yield was 91.0% and the purity was 83.21%.

### Example 5:

### Preparation of 5-(α-cyclopropylcarbonyl-2-fluorobenzyl)-2-oxo -4,5,6,7-tetrahydrothieno[3,2-c]pyridine (compound 4)

25.7g α-cyclopropylcarbonyl-2-fluorobenzyl chloride prepared in Example 2, 47.5g 2-oxo-4,5,6,7-tetrahydrothieno[3,2-c]pyridine p-toluenesulfonate, 40.6g sodium bicarbonate, 0.95g potassium bromide, 2.6g tetrabutylammonium bromide and 180ml DMF were added to a 1000ml four-neck flask equipped with a mechanical stirring device, a thermometer, a reflux condenser and a constant-pressure dropping funnel. The mixture was stirred for 45 minutes under nitrogen atmosphere at 40°C∼55°C and cooled. 220ml ethyl acetate and 350ml water were added thereto, and the system was allowed to seperation. The organic layer was washed with 350ml water and 120ml saturated brine successively, and then dried over anhydrous magnesium sulfate. The resulting mixture was decolored with silica gel bed and filtered. The filtrate was distillated under reduced pressure and concentrated to give 38.45g brown gum. The yield was 72.3% and the purity was 86.45%.

### Example 6:

### Preparation of 2-acetyloxy-5-(α-cyclopropylcarbonyl-2-fluorobenzyl) -4,5,6,7-tetrahydrothieno[3,2-c]pyridine (prasugrel)

18.76g 5-(α-cyclopropylcarbonyl-2-fluorobenzyl)-2-oxo -4,5,6,7-tetrahydrothieno[3,2-c]pyridine (compound 4) prepared in Example 3, 4 or 5 and 60ml DMF were added to a 500ml four-neck flask equipped with a mechanical stirring device, a thermometer and a constant-pressure dropping funnel. The flask was placed in a water-ice bath and 17.17g triethylamine was injected under nitrogen atmosphere. The temperature was controlled at -10°C to 5°C and 17.34g acetic anhydride was added slowly in dropwise over 30 minutes. After the completion of addition, the mixture was kept for 10 minutes at this temperature and then stirred for 2 hours at room temperature. 100ml ethyl acetate and 150ml water were added thereto, and the system was allowed to seperation. The organic layer was washed with 150ml water, 50ml saturated NaHCO₃ and 50ml saturated brine successively, and then dried over anhydrous magnesium sulfate. The resulting mixture was decolored with silica gel bed and filtered. The filtrate was distillated under reduced pressure and concentrated to give 26.10g brown gum.

### Example 7

26.10g prasugrel gum prepared in Example 6 was added to 70ml ethanol. The mixture was stirred for 12 hours at room temperature under nitrogen atmosphere, crystallized and filtered to give 12.2g off-white solid. The yield was 56.0% and the purity was 97.02%. The product was recrystallized from 33ml ethanol to give 10.6g white solid. The melting point was 123°C and the purity was 99.75%.

### Example 8

26.1g prasugrel gum prepared in Example 6 was added to 100ml isopropanol. The mixture was stirred for 12 hours at room temperature under nitrogen atmosphere, crystallized and filtered to give 10.5g off-white solid. The yield was 48.2% and the purity was 99.54%. The product was recrystallized from 42ml isopropanol to give 9.0g white solid. The melting point was 122°C and the purity was 99.80%.

The present invention has been described in detail hereinbefore, including its preferred embodiments.

## Claims

1. A method for synthesizing crystalline form A of prasugrel,
wherein the crystalline form A has the following X-ray powder diffraction pattern measured by copper cathode diffractometer, expressed as the interplanar spacing d, Bragg angle 2θ, intensity and relative intensity, wherein the relative intensity is expressed as a percentage relative to the strongest ray:
| 2θ angle (°) | interplanar spacing d(Å) | intensity | relative intensity (%) |
|---|---|---|---|
| 7.639 | 11.56 | 1569 | 19 |
| 9.717 | 9.09 | 192 | 2.3 |
| 11.118 | 7.95 | 2838 | 34.4 |
| 13.340 | 6.63 | 8253 | 100 |
| 13.679 | 6.47 | 808 | 9.8 |
| 14.360 | 6.16 | 4184 | 50.7 |
| 14.639 | 6.05 | 5807 | 70.4 |
| 14.980 | 5.91 | 2125 | 25.7 |
| 17.737 | 5.00 | 720 | 8.7 |
| 18.480 | 4.80 | 2087 | 25.3 |
| 18.761 | 4.73 | 8089 | 98 |
| 19.221 | 4.61 | 6421 | 77.8 |
| 20.640 | 4.30 | 707 | 8.6 |
| 21.301 | 4.17 | 7910 | 95.8 |
| 22.298 | 3.98 | 1681 | 20.4 |
| 22.640 | 3.92 | 1743 | 21.1 |
| 23.300 | 3.81 | 7209 | 87.4 |
| 24.258 | 3.67 | 6855 | 83.1 |
| 24.682 | 3.60 | 649 | 7.9 |
| 26.221 | 3.40 | 1237 | 15 |
| 26.862 | 3.32 | 1947 | 23.6 |
| 28.320 | 3.15 | 501 | 6.1 |
| 29.422 | 3.03 | 548 | 6.6 |
| 30.179 | 2.96 | 323 | 3.9 |
| 31.280 | 2.86 | 2091 | 25.3 |
| 31.922 | 2.80 | 632 | 7.7 |
| 32.780 | 2.73 | 924 | 11.2 |
| 33.421 | 2.68 | 362 | 4.4 |
| 34.960 | 2.56 | 531 | 6.4 |
| 35.557 | 2.52 | 218 | 2.6 |
| 36.960 | 2.43 | 486 | 5.9 |
| 38.060 | 2.36 | 583 | 7.1 |
| 38.339 | 2.35 | 358 | 4.3 |
| 39.000 | 2.31 | 953 | 11.5 |
**characterized in that** the method comprises the following steps:
(1) o-fluorobenzyl cyclopropyl ketone as a raw material is reacted with 1,3-dibromo-5,5-dimethylhydantoin or 1,3-dichloro-5,5- dimethylhydantoin as a halogenating agent in acetic acid as a solvent to give α-cyclopropylcarbonyl-2-fluorobenzyl halide (compound 2);
(2) α-cyclopropylcarbonyl-2-fluorobenzyl halide is reacted with 2-oxo-4,5,6,7-tetrahydrothieno[3,2-c]pyridine toluenesulfonate (compound 3) in the concerted catalysis of a phase transfer catalyst and an inorganic salt using an inorganic base as an acid binding agent and DMF as a solvent under nitrogen atmosphere to get 5-(α-cyclopropylcarbonyl-2-fluorobenzyl)-2-oxo-2,4,5,6,7-tetrahydrothieno[3,2-c]pyridi ne (compound 4);
(3) 5-(α-cyclopropylcarbonyl-2-fluorobenzyl)-2-oxo-2,4,5,6,7-tetrahydrothieno[3,2-c]pyridine is acylated in the presence of an acylating reagent using triethylamine as an acid binding agent and DMF as a solvent under nitrogen atmosphere at room temperature, and finally extracted, dried and filtrated to give the mother liquor of prasugrel, and the mother liquor is concentrated to get prasugrel as a gum;
(4) an alcoholic solvent is added to the prasugrel as a gum obtained in the above step, and crystal is precipitated after stirring at room temperature, then the crystal is filtrated to give prasugrel as solid;
the reaction scheme is as follows:

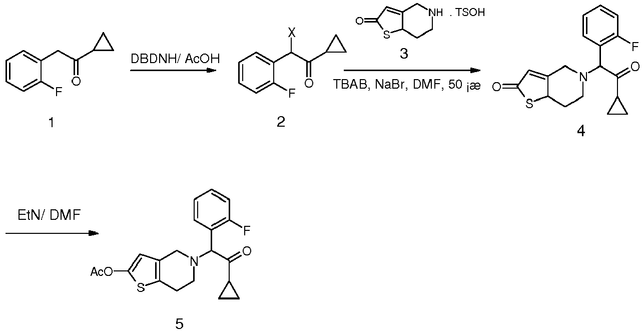

2. The synthesis method according to claim 1, **characterized in that** the molar ratio of o-fluorobenzyl cyclopropyl ketone to the halogenating agent is 1:0.5-0.65.

3. The synthesis method according to claim 1, **characterized in that** the molar ratio of α-cyclopropylcarbonyl-2-fluorobenzyl bromide to the inorganic base in step (2) is 1:2-8.

4. The synthesis method according to claim 1 or 3, **characterized in that** the inorganic base is one or more selected from the group consisting of sodium carbonate, potassium carbonate, sodium bicarbonate, and potassium bicarbonate.

5. The synthesis method according to claim 1, **characterized in that** the molar ratio of α-cyclopropylcarbonyl-2-fluorobenzyl bromide to the phase transfer catalyst in step (2) is 1:0.05-1.2.

6. The synthesis method according to claim 1 or 5, **characterized in that** the phase transfer catalyst is any one selected from the group consisting of tetrabutylammonium bromide, tetraethyl ammonium bromide, tetrabutyl ammonium chloride and tetraethylammonium chloride.

7. The synthesis method according to claim 1, **characterized in that** the molar ratio of α-cyclopropylcarbonyl-2-fluorobenzyl bromide to the inorganic salt in step (2) is 1:0.02-1.2.

8. The synthesis method according to claim 1 or 7, **characterized in that** the inorganic salt is one selected from the group consisting of sodium bromide and potassium bromide.

9. The synthesis method according to claim 1, **characterized in that** the molar ratio of 5-(α-cyclopropylcarbonyl-2-fluorobenzyl)-2-oxo-2,4,5,6,7 -tetrahydrothieno[3,2-c]pyridine to triethylamine in step (3) is 1:1∼10.

10. The synthesis method according to claim 1, **characterized in that** the alcoholic solvent in step (4) is any one selected from the group consisting of methanol, ethanol, isopropanol, n-butanol, tert-butanol, benzyl alcohol and benzoic alcohol, or any mixture thereof.

11. The synthesis method according to claim 1 or 10, **characterized in that** the ratio of the volume of the alcoholic solvent to the weight of α-cyclopropylcarbonyl-2-fluorobenzyl halide is 2.5ml/g∼6.5ml/g.

## Patentansprüche

1. Verfahren zur Synthese der kristallinen Form A von Prasugrel,
wobei die kristalline Form A das nachfolgende mit Kupferkathodendiffraktometer gemessene, als interplanarer Abstand d, Bragg-Winkel 2θ, Intensität und relative Instität ausgedrückte Röntgen-Pulverdiffraktometriemuster aufweist, wobei die relative Intensität als Prozentanteil bezogen auf den stärksten Strahl ausgedrückt wird:
| 2θ-Winkel (°) | interplanarer Abstand d(A) | Intensität | Relative Intensität (%) |
|---|---|---|---|
| 7,639 | 11,56 | 1569 | 19 |
| 9,717 | 9,09 | 192 | 2,3 |
| 11,118 | 7,95 | 2838 | 34,4 |
| 13,340 | 6,63 | 8253 | 100 |
| 13,679 | 6,47 | 808 | 9,8 |
| 14,360 | 6,16 | 4184 | 50,7 |
| 14,639 | 6,05 | 5807 | 70,4 |
| 14,980 | 5,91 | 2125 | 25,7 |
| 17,737 | 5,00 | 720 | 8,7 |
| 18,480 | 4,80 | 2087 | 25,3 |
| 18,761 | 4,73 | 8089 | 98 |
| 19,221 | 4,61 | 6421 | 77,8 |
| 20,640 | 4,30 | 707 | 8,6 |
| 21,301 | 4,17 | 7910 | 95,8 |
| 22,298 | 3,98 | 1681 | 20,4 |
| 22,640 | 3,92 | 1743 | 21,1 |
| 23,300 | 3,81 | 7209 | 87,4 |
| 24,258 | 3,67 | 6855 | 83,1 |
| 24,682 | 3,60 | 649 | 7,9 |
| 26,221 | 3,40 | 1237 | 15 |
| 26,862 | 3,32 | 1947 | 23,6 |
| 28,320 | 3,15 | 501 | 6,1 |
| 29,422 | 3,03 | 548 | 6,6 |
| 30,179 | 2,96 | 323 | 3,9 |
| 31,280 | 2,86 | 2091 | 25,3 |
| 31,922 | 2,80 | 632 | 7,7 |
| 32,780 | 2,73 | 924 | 11,2 |
| 33,421 | 2,68 | 362 | 4,4 |
| 34,960 | 2,56 | 531 | 6,4 |
| 35,557 | 2,52 | 218 | 2,6 |
| 36,960 | 2,43 | 486 | 5,9 |
| 38,060 | 2,36 | 583 | 7,1 |
| 38,339 | 2,35 | 358 | 4,3 |
| 39,000 | 2,31 | 953 | 11,5 |
**dadurch gekennzeichnet, dass** das Verfahren die nachfolgenden Schritte umfasst:
(1) o-Fluorbenzylcyclopropylketon als Rohstoff wird mit 1,3-Dibrom-5,5-dimethylhydantoin oder 1,3-Dichlor-5,5- dimethylhydantoin als Halogeniermittel in Essigsäure als Lösemittel reagiert, um α-Cyclopropylcarbonyl-2-fluorbenzylhalogenid (Verbindung 2) zu ergeben;
(2) α-Cyclopropylcarbonyl-2-fluorbenzylhalogenid wird mit 2-Oxo-4,5,6,7-tetrahydrothieno[3,2-c]pyridintoluolsulfonat (Verbindung 3) in der abgestimmten Katalyse eines Phasentransferkatalysators und eines anorganischen Salzes unter Verwendung einer anorganischen Base als Säurebindemittel und DMF als Lösemittel unter Stickstoffatmosphäre reagiert, um 5-(α-Cyclopropylcarbonyl-2-fluorbenzyl)-2-oxo-2,4,5,6,7-tetrahydrothieno[3,2-c]pyridin (Verbindung 4) zu ergeben;
(3) 5- (α-Cyclopropylcarbonyl-2-fluorbenzyl)-2-oxo-2,4,5,6,7-tetrahydrothieno[3,2-c]pyridin wird in Gegenwart eines acylierenden Reagenzes mit Triethylamin als Säurebindemittel und DMF als Lösemittel unter Stickstoffatmosphäre bei Raumtemperatur acyliert und schließlich extrahiert, getrocknet und filtriert, um die Mutterlauge von Prasugrel zu ergeben, und die Mutterlauge wird konzentriert, um Prasugrel als Gummi zu ergeben;
(4) ein alkoholisches Lösemittel wird dem aus dem obigen Schritt als Gummi resultierenden Prasugrel hinzugegeben und Kristall wird nach dem Rühren bei Raumtemperatur ausgefällt, dann wird der Kristall filtriert, um Prasugrel als Festkörper zu ergeben;
das Reaktionsschema ist wie folgt:

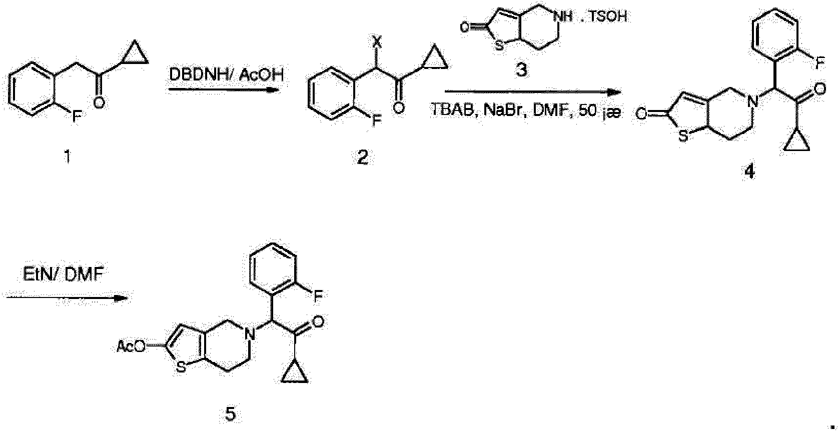

2. Syntheseverfahren nach Anspruch 1, **dadurch gekennzeichnet, dass** das Molverhältnis von o-Fluorbenzylcyclopropylketon zum Halogeniermittel 1: 0,5 ∼ 0,65 beträgt.

3. Syntheseverfahren nach Anspruch 1, **dadurch gekennzeichnet, dass** das Molverhältnis von α-Cyclopropylcarbonyl-2-fluorbenzylbromid zur anorganischen Base im Schritt (2) 1: 2 ∼ 8 beträgt.

4. Syntheseverfahren nach Anspruch 1 oder 3, **dadurch gekennzeichnet, dass** die anorganische Base mindestens eine ist, die aus der nachfolgenden Gruppe gewählt ist: Natriumcarbonat, Kaliumcarbonat, Natriumhydrogencarbonat und Kaliumhydrogencarbonat.

5. Syntheseverfahren nach Anspruch 1, **dadurch gekennzeichnet, dass** das Molverhältnis von α-Cyclopropylcarbonyl-2-fluorbenzylbromid zum Phasentransferkatalysator im Schritt (2) 1: 0,05 ∼ 1,2 beträgt.

6. Syntheseverfahren nach Anspruch 1 oder 5, **dadurch gekennzeichnet, dass** der Phasentransferkatalysator aus der nachfolgenden Gruppe gewählt ist: Tetrabutylammoniumbromid, Tetraethylammoniumbromid, Tetrabutylammoniumchlorid und Tetraethylammoniumchlorid.

7. Syntheseverfahren nach Anspruch 1, **dadurch gekennzeichnet, dass** das Molverhältnis von α-Cyclopropylcarbonyl-2-fluorbenzylbromid zum anorganischen Salz im Schritt (2) 1: 0,02 ∼ 1,2 beträgt.

8. Syntheseverfahren nach Anspruch 1 oder 7, **dadurch gekennzeichnet, dass** das anorganische Salz aus der nachfolgenden Gruppe gewählt ist: Natriumbromid und Kaliumbromid.

9. Syntheseverfahren nach Anspruch 1, **dadurch gekennzeichnet, dass** das Molverhältnis von 5-(α-Cyclopropylcarbonyl-2-fluorbenzyl)-2-oxo-2,4,5,6,7-tetrahydrothieno[3,2-c]pyridin zu Triethylamin im Schritt (3) 1: 1∼10 beträgt.

10. Syntheseverfahren nach Anspruch 1, **dadurch gekennzeichnet, dass** das alkoholische Lösemittel im Schritt (4) aus der nachfolgenden Gruppe gewählt ist: Methanol, Ethanol, Isopropanol, n-Butanol, tert-Butanol, Benzylalkohol und Benzoealkohol oder eine Mischung davon.

11. Syntheseverfahren nach Anspruch 1 oder 10, **dadurch gekennzeichnet, dass** das Verhältnis des Volumens des alkohlischen Lösemittels zum Gewicht des α-Cyclopropylcarbonyl-2-fluorbenzylhalogenids 2,5 ml/g ∼ 6,5 ml/g beträgt.

## Revendications

1. Procédé de synthèse de la forme cristalline A du prasugrel,
dans lequel la forme cristalline A présente le spectre de diffraction des rayons X sur poudre suivant mesuré par diffractomètre à cathode de cuivre, exprimé par l'espacement interplanaire d, l'angle Bragg de 2θ, l'intensité et l'intensité relative, dans lequel l'intensité relative est exprimée par un pourcentage par rapport au rayonnement le plus intense :
| angle 2θ (°) | espacement interplanaire d(Å) | intensité | intensité relative (%) |
|---|---|---|---|
| 7,639 | 11,56 | 1 569 | 19 |
| 9,717 | 9,09 | 192 | 2,3 |
| 11,118 | 7,95 | 2 838 | 34,4 |
| 13,340 | 6,63 | 8 253 | 100 |
| 13,679 | 6,47 | 808 | 9,8 |
| 14,360 | 6,16 | 4 184 | 50,7 |
| 14,639 | 6,05 | 5 807 | 70,4 |
| 14,980 | 5,91 | 2 125 | 25,7 |
| 17,737 | 5,00 | 720 | 8,7 |
| 18,480 | 4,80 | 2 087 | 25,3 |
| 18,761 | 4,73 | 8 089 | 98 |
| 19,221 | 4,61 | 6 421 | 77,8 |
| 20,640 | 4,30 | 707 | 8,6 |
| 21,301 | 4,17 | 7 910 | 95,8 |
| 22,298 | 3,98 | 1 681 | 20,4 |
| 22,640 | 3,92 | 1 743 | 21,1 |
| 23,300 | 3,81 | 7 209 | 87,4 |
| 24,258 | 3,67 | 6 855 | 83,1 |
| 24,682 | 3,60 | 649 | 7,9 |
| 26,221 | 3,40 | 1 237 | 15 |
| 26,862 | 3,32 | 1 947 | 23,6 |
| 28,320 | 3,15 | 501 | 6,1 |
| 29,422 | 3,03 | 548 | 6,6 |
| 30,179 | 2,96 | 323 | 3,9 |
| 31,280 | 2,86 | 2 091 | 25,3 |
| 31,922 | 2,80 | 632 | 7,7 |
| 32,780 | 2,73 | 924 | 11,2 |
| 33,421 | 2,68 | 362 | 4,4 |
| 34,960 | 2,56 | 531 | 6,4 |
| 35,557 | 2,52 | 218 | 2,6 |
| 36,960 | 2,43 | 486 | 5,9 |
| 38,060 | 2,36 | 583 | 7,1 |
| 38,339 | 2,35 | 358 | 4,3 |
| 39,000 | 2,31 | 953 | 11,5 |
**caractérisé en ce que** le procédé comprend les étapes suivantes :
(1) de l'o-fluorobenzyl cyclopropyl cétone en tant que matière première est mise à réagir avec de la 1,3-dibromo-5,5-diméthylhydantoïne ou de la 1,3-dichloro-5,5-diméthylhydantoïne en tant qu'agent d'halogénation dans de l'acide acétique en tant que solvant pour donner un halogénure d'α-cyclopropylcarbonyl-2-fluorobenzyle (composé 2) ;
(2) l'halogénure α-cyclopropylcarbonyl-2-fluorobenzyle est mis à réagir avec du toluènesulfonate de 2-oxo-4,5,6,7-tétrahydrothiéno[3,2-c]pyridine (composé 3) dans la catalyse conjointe d'un catalyseur de transfert de phase et d'un sel inorganique à l'aide d'une base inorganique en tant qu'agent de liaison aux acides et de DMF en tant que solvant dans une atmosphère azotée pour obtenir de la 5-(α-cyclopropylcarbonyl-2-fluorobenzyl)-2-oxo-2,4,5,6,7-tétrahydrothiéno[3,2-c]pyridine (composé 4) ;
(3) la 5-(α-cyclopropylcarbonyl-2-fluorobenzyl)-2-oxo-2,4,5,6,7-tétrahydrothiéno[3,2-c]pyridine est acylée en présence d'un réactif acylant à l'aide de triéthylamine en tant qu'agent de liaison aux acides et de DMF en tant que solvant dans une atmosphère azotée à température ambiante, et enfin extraite, séchée et filtrée pour donner la liqueur mère de prasugrel, et la liqueur mère est concentrée pour obtenir le prasugrel sous la forme d'une gomme ;
(4) un solvant alcoolique est ajouté au prasugrel sous forme de gomme obtenu dans l'étape ci-dessus, et un cristal est précipité après agitation à température ambiante, puis le cristal est filtré pour donner le prasugrel sous la forme d'un solide ;
le schéma réactionnel est comme suit :

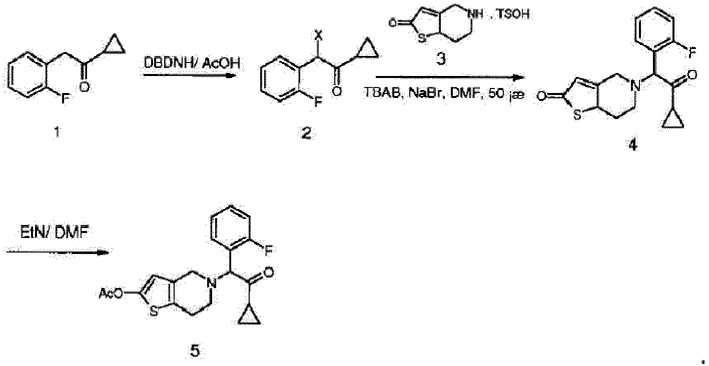

2. Procédé de synthèse selon la revendication 1, **caractérisé en ce que** le rapport molaire de l'o-fluorobenzyl cyclopropyl cétone à l'agent d'halogénation est de 1 :0,5∼0,65.

3. Procédé de synthèse selon la revendication 1, **caractérisé en ce que** le rapport molaire du bromure d'α-cyclopropylcarbonyl-2-fluorobenzyle à la base inorganique dans l'étape (2) est de 1 :2∼8.

4. Procédé de synthèse selon la revendication 1 ou 3, **caractérisé en ce que** la base inorganique est un ou plusieurs composés choisis dans le groupe constitué du carbonate de sodium, du carbonate de potassium, du bicarbonate de sodium et du bicarbonate de potassium.

5. Procédé de synthèse selon la revendication 1, **caractérisé en ce que** le rapport molaire du bromure d'α-cyclopropylcarbonyl-2-fluorobenzyle au catalyseur de transfert de phase dans l'étape (2) est de 1 :0,05∼1,2.

6. Procédé de synthèse selon la revendication 1 ou 5, **caractérisé en ce que** le catalyseur de transfert de phase est l'un quelconque des composés choisis dans le groupe constitué du bromure de tétrabutylammonium, du bromure de tétraéthylammonium, du chlorure de tétrabutylammonium et du chlorure de tétraéthylammonium.

7. Procédé de synthèse selon la revendication 1, **caractérisé en ce que** le rapport molaire du bromure d'α-cyclopropylcarbonyl-2-fluorobenzyle au sel inorganique dans l'étape (2) est de 1 :0,02∼1,2.

8. Procédé de synthèse selon la revendication 1 ou 7, **caractérisé en ce que** le sel inorganique est un composé choisi dans le groupe constitué du bromure de sodium et du bromure de potassium.

9. Procédé de synthèse selon la revendication 1, **caractérisé en ce que** le rapport molaire de la 5-(α-cyclopropylcarbonyl-2-fluorobenzyl)-2-oxo-2,4,5,6,7-tétrahydrothiéno[3,2-c]pyridine à la triéthylamine dans l'étape (3) est de 1 :1∼10.

10. Procédé de synthèse selon la revendication 1, **caractérisé en ce que** le solvant alcoolique dans l'étape (4) est l'un quelconque des composés choisis dans le groupe constitué du méthanol, de l'éthanol, de l'isopropanol, du n-butanol, du tert-butanol, de l'alcool benzylique et de l'alcool benzoïque, ou de tout mélange de ceux-ci.

11. Procédé de synthèse selon la revendication 1 ou 10, **caractérisé en ce que** le rapport du volume du solvant alcoolique au poids de l'halogénure α-cyclopropylcarbonyl-2-fluorobenzyle est de 2,5 ml/g à 6,5 ml/g.
